# EUROPEAN PATENT SPECIFICATION

(11) **EP 1 344 451 B1**
(45) Date of publication and mention of the grant of the patent: **21.12.2005**
(21) Application number: 03075205.9
(22) Date of filing: 23.01.2003
(51) Int. Cl.: A01K 29/00, A61B 5/05, A61B 5/107, A01J 5/013, A01J 5/007

(54) **A device for an animal related action**
Vorrichtung für eine tierbezogene Handlung
Appareil pour exercer une action relative à un animal

(30) Priority: 11.03.2002 NL 1020148
(43) Date of publication of application: 17.09.2003
(60) Divisional application: 05077377.9
(73) Proprietor: Lely Enterprises AG, 6300 ZUG (CH)
(72) Inventor: Van den Berg, Karel, 2971 BR Bleskensgraaf (NL); Aarts, Adrianus Petrus Hendrikus, 4714 BG Sprundel (NL); Theelen, Antoon Peter André, 3142 CM Maassluis (NL); van Lenteren, Adriaan Coert, 3155 RH Maasland (NL)
(74) Representative: Corten, Maurice Jean F.M.

(56) References cited:
- WO-A-00/38502
- WO-A-96/04551
- WO-A-99/33022
- GB-A- 1 398 735
- US-B1- 6 208 890

## Description

The invention relates to a device for automatically measuring a characteristic of the body of a living animal according to the preamble of claim 1.

Such a device is known from WO 96/04551.

In general, the body-score of an animal is considered as a good measure for the condition of health and the production capacity of an animal. Although there does exist a relation between the weight and the body-score, said body-score can not, or very badly, be determined on the basis of the weight of the animal. The fact is that the weight is also influenced by many other factors, such as the degree to which the stomach is filled, the weight of the contents of the bladder and the bowel of the animal, or the weight of a possible foetus. These factors may even vary during the day and disturb a good determination of the body-score.

It is a first object of the invention to provide an alternative device of the above-mentioned type by means of which an accurate determination of the body-score is possible.

To this end, a device of the above-described type according to the invention is characterized by the features of claim 1. The fat content of an animal, in particular a cow, is an important characteristic for the determination of its condition of health. There is a direct relation with the body-score of the animal. If this bag is disposed on the back of the animal to be measured, the bag will assume the shape of the profile of the back. The total outer surface of the bag remains equal to the outer surface of the bag in its condition of rest. However, its volume will change. An advantage of this profile measurement is that it can be carried out in a cheap and robust manner.

In a special embodiment, the profile meter comprises a stop for keeping the profile meter in a fixed position relative to the body of the animal. In this way the surface related contents of the bag, which is a measure for the profile, is always determined relative to the same position of an animal. Consequently, different profile measurements of an animal can be compared with each other in a reliable manner.

It is advantageous if the fluid measuring device comprises a volume meter. Volume changes can be measured in a relatively simple and cheap manner.

In another advantageous embodiment, the fluid measuring device comprises a flow meter. By means of said flow meter it is possible accurately to indicate changes in the profile when the bag is moved over a profile.

In a particular embodiment according to the invention, there is provided a heating element in the fluid for keeping the fluid at a constant temperature. A constant temperature in the region of the body temperature of the animal is necessary for making the contact with the animal as pleasant as possible. Moreover, by means of this measure it is possible to prevent the fluid from freezing or coagulating.

The fluid is in particular a liquid because of the fact that liquids can be measured more easily than gases.

In a particular embodiment, the device comprises means for automatically performing an animal related action. In this way it is possible also to determine the fat content of animals that are present in the device for undergoing the animal related action. It is thus possible to combine actions for the measurement and actions for the animal related action, so that the animal will experience less stress.

In a further embodiment according to the invention, the device comprises an animal recognition device for recognising individual animals. By means of the animal recognition device it is possible to link the measured fat content to a particular individual animal.

In a further advantageous embodiment, the determining means are provided with a processing device with a memory, the processing device comprising a data table for determining the fat content by means of the measurement signal and the processing device being suitable for issuing a fat content signal that is indicative of the determined fat content. For a correct determination of the fat content from the measurement signal obtained, there are required more data, such as breed, age, fat content measured in the past, environmental factors, etc. These factors are stored in a data table. Data from said table can be used in an algorithm that processes the measurement signal for determining the correct fat content. The data table may also comprise data per group of animals. It frequently occurs that a group of animals gives a response that is more or less the same. By comparing the measurement signal with a value of a group of animals from the data table, it is possible to allot to the relevant animal the fat content signal of the corresponding group of animals. The data table may contain data per individual animal. Each animal is different and, therefore, individual data of an animal will be required for an accurate determination of the fat content.

In a further embodiment, the processing device comprises a comparing device for comparing a momentary fat content determined from a momentary measurement signal with a historical fat content. This makes it possible to indicate whether there is a change in the fat content of an animal or a group of animals. This information is important for a good management of the animal or a group of animals. In some cases said change will be desired; in other cases said change will be undesired because it is an indication of illness or incorrect feeding. In the latter case measures can be taken to adjust the body-score.

In an embodiment according to the invention, the device comprises an indicating device for indicating the fat content signal. In this way it is possible for an operator to be informed of the fat content of an animal at the location of the device or remotely via a terminal or a mobile telephone.

In another embodiment according to the invention, the device comprises a warning device for generating a warning, which warning device can be controlled by the fat content signal. In this way it is possible for the operator or a controlling computer to be warned, at the location of the device or remotely, if for example a particular threshold of the fat content signal has been exceeded.

In a further embodiment according to the invention, the device can be controlled by the fat content signal. Depending on the body-score of an animal, the animal has to be treated in a particular manner. In the currently applied management systems, the farmer judges the body-score on the basis of visual observations and palpations carried out on the cow, completed by external data, if any, such as milk analysis reports. According to the judgement of the body-score, the treatment will be adapted. However, this is very labour-intensive and the actual adaptation often takes place later than desired. By immediately adapting the animal related action in the device to the momentary condition of health or production condition in the form of the body-score, labour is saved and an immediate reaction to a change is possible.

In a further embodiment, the device comprises a feeding installation for supplying feed. The momentary body-score can be controlled in the direction of the desired body-score by adapting the feed to be supplied. For example, to a cow with a too high body-score, less concentrate or concentrate having a lower energy content should be supplied in the feed trough of the device, in order to reduce her body-score.

In a further advantageous embodiment, the device comprises a milking machine for automatically performing on the dairy animal a milking related action, such as stimulation, foremilking, pre-cleaning, main milking, post-cleaning and the like. In this way the fat content can be determined automatically at each milking run of the dairy animal. In that case this needs not to be carried out separately by means of a separate installation. If desired, after the momentary production capacity has been determined on the basis of the fat content, the milking machine can be adapted immediately to this new production capacity.

In an advantageous embodiment, an electromagnetic parameter of the animal can be measured by the measuring means. By measuring said parameter, the use of measuring means comprising few if any moving parts will suffice. This results in a robust embodiment. In particular in an aggressive environment, such as an animal-environment, this is important.

It is advantageous if the measuring means comprise a TOBEC (total body electrical conductivity) meter. This type of meter measures the fat contact in a contactless way, the animal not being hindered by the measurement. These are important conditions for an animal-friendly cattle breeding. An example of such a meter is disclosed in British patent GB1398735.

In another embodiment, the measuring means comprise an electrical impedance meter. Measurement of the electrical impedance can be carried out by means of relatively simple and cheap equipment. Because the measurement is carried out whilst using small currents and low voltages, this measurement does not lead to malfunction of electronically sensitive equipment that may be present around the animal.

In a particular embodiment, the impedance meter comprises an udder electrode for contact with the udder of the animal. In another particular embodiment, the impedance meter comprises a snout electrode for contact with the snout of the animal. Both the snout and the udder are hardly or not covered with hair and can ensure a good electrical conductivity contact with an electrode without further measures being taken.

It is advantageous to dispose the udder electrode in or on a teat cup. Because, for the purpose of milking the animal, a teat cup is connected to the teat of the animal, no extra mechanical provisions, especially for disposing an electrode on the udder, need to be made.

In a special embodiment, the snout electrode is disposed in or on the feed trough of the feeding installation. During the consumption of feed from the feed trough, the snout of the animal will nearly always make contact with the feed trough. It is extra advantageous that the contact point is situated rather constantly at the same place.

According to the invention, the measuring means comprise a profile meter for measuring at least a part of the profile of the animal. By measuring the profile of the animal and comparing it with standard profiles, the body-score can be reduced.

The profile preferably comprises the profile of the upper side of the animal. The condition of the animal is most strongly expressed in the profile of the back of the animal. Apart from this, the upper side of the animal can easily be measured, because it is usually freely accessible from above.

In a preferred embodiment according to the invention, the profile comprises the profile of the tail portion. Reserve fat is especially stored in the tail portion, so that the profile of the tail portion is a good indicator of the body-score. The tail portion comprises roughly the area around the lumbar vertebras, the ischia and the beginning of the caudal vertebras.

In a further preferred embodiment, the profile comprises the profile of the shoulder. The shoulder is a place where much reserve fat is stored. An advantage of the shoulder is that it has a round, rather regular shape and that it can easily be measured.

In another embodiment, the profile meter comprises a camera that is connected to a picture-analysing device. This has the advantage that the camera, apart from measuring the profile, can also serve as security camera.

In a specific embodiment, the profile meter comprises a contactless telemeter for measuring the distance from the meter to the surface of the animal. In this way small differences in height in the profile can be detected accurately. This is in particular important for determining the precise shape of the cavities in the area in the vicinity of the tailhead, pin bone, thurl and hip bone. The shape of said cavities is strongly determinative for the body-score. The contactless telemeter can be designed as a laser-Doppler meter or an ultrasonic meter.

In an embodiment, but not according to the invention, the profile meter comprises one or more feeler devices. A measurement by means of feeler devices is not disturbed by the effect of shadow or the reflection condition of the skin. Due to this, the measurement is less sensitive to environmental factors.

In an embodiment according to the invention, the profile meter is connected to a following device for following movements of the animal. For this purpose, the profile meter follows for example the device for following the rear of the animal or a robot arm of the milking machine. Thus the measurement is not disturbed by movements of the animal.

It is advantageous if the profile meter is movable relative to the animal. This makes it possible to determine the profile of a large surface in an accurate and controlled manner.

A second device for an animal related action can be controllable by the control signal. It frequently occurs that a certain amount of feed is supplied in the device for the animal related action. The latter device is optimally utilised if it is only kept occupied for the animal related action. Then, the planned amount of feed to be supplied is not always consumed. In that case it is advantageous if the remaining amount can be supplied in a second feeding installation that is only suitable for supplying feed.

The invention will be explained hereinafter in detail with reference to drawings of exemplary embodiments.
Figure 1 is a plan view of an embodiment according to the invention with a cow present therein.
Figure 2 is a detail of the rear side of an embodiment, but not according to the invention provided with a profile meter in the form of feeler devices
Figure 3 is a detail of the rear side of a device according to the invention provided with a profile meter in the form of a bag.

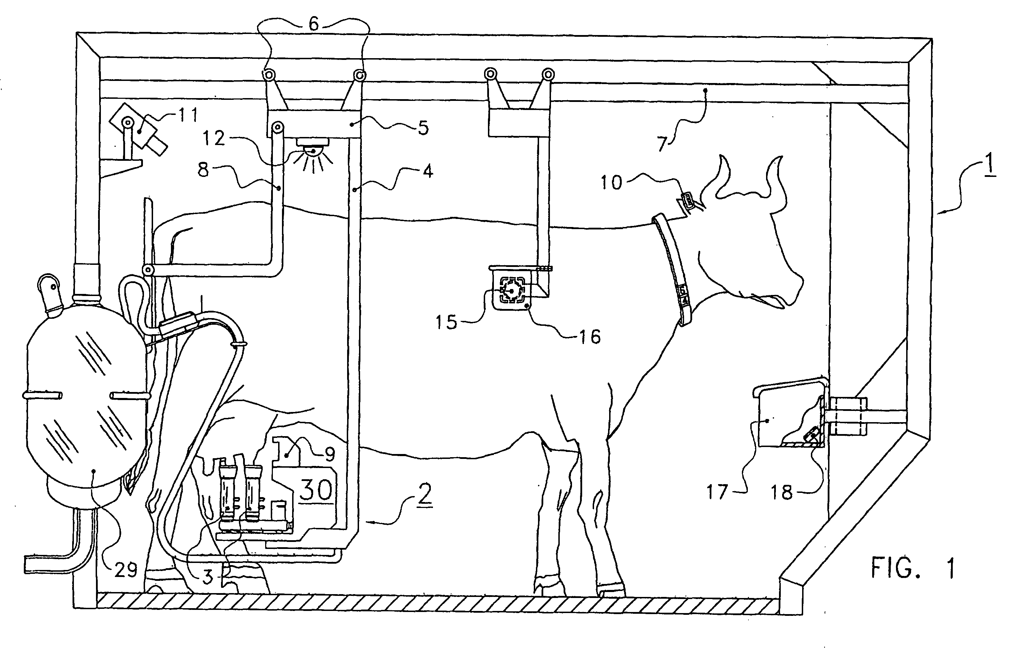

Figure 1 is an overall picture of an embodiment of the device. The device is included in a non-shown cow area, such as a shed, where cows can move about freely. The basis of the device is constituted by a box-shaped frame 1 that serves as fastening place for appendages and for positioning a cow to be milked. A robot arm 2 with milking equipment is provided in the device for automatically connecting teat cups 3 to the udder of the cow. The robot arm 2 is connected, via a vertical connecting element 4, to an arm suspension 5. By means of a drive and wheels 6, said arm suspension 5 is movable over a rail 7 that constitutes part of the frame 1. A device 8 for following the rear of the animal is fitted to the arm suspension 5. Said device 8 for following the rear of the animal is continuously in contact with the rear of the animal, so that movements of the animal in the longitudinal direction of the device are observed by sensors and the drive of the arm suspension 5 can be controlled accordingly. Teat cups 3 are connected to the arm for the purpose of milking the animal. The teat cups 3 can be connected automatically to the teats. There is provided a laser 9 for locating the teats. There is provided a milk glass 29 for collecting the milk from the teat cups before discharging the milk to a storage unit. Each cow is provided with a transponder 10 so as to be able to be identified by the device.

A camera 11 is disposed on the frame 1 for taking a picture of the animal. The camera 11 is focussed in particular on the rear part of the body. The camera 11 is disposed movably, so that the position in which the picture is taken can be adapted to the dimensions of the animal. A computer is connected to the camera 11 for analysing the pictures taken. The picture-analysing program of the computer recognises in each digital picture characteristic points of the animal, such as the position of the hip bones and the spine. Said points are used for determining the profile.

In a non-shown embodiment, the profile is determined with the aid of the shadow of the animal as described in the international application WO99/33022.

An ultrasonic meter 12 is fastened to the arm suspension 5 so as to be positioned above the back of the animal. Because the arm suspension 5 follows the cow, the ultrasonic meter 12 will always be positioned above a specific place on the back. The ultrasonic meter 12 measures the distance from the meter to a point on the back by emitting and receiving ultrasonic sound waves. The profile is determined by measuring the distance to several points.

Instead of an ultrasonic meter, a laser-Doppler meter may be used. The principle of operation remains the same: instead of sound waves, laser beams are emitted in the direction of the animal, reflected and received again. The distance is determined from the comparison of the emitted beams with the reflected beams. In particular the depth of the cavities in the area in the vicinity of the tailhead, pin bone, thurl and hip bone constitutes an important value.

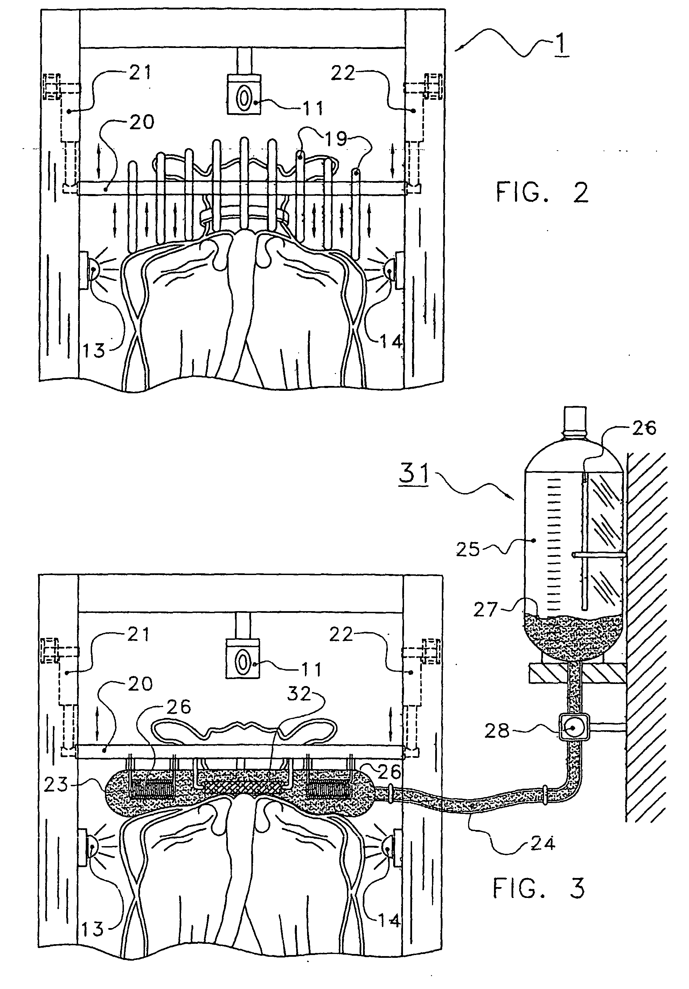

In Figure 2 a laser-Doppler meter 13 and an ultrasonic meter 14 are fastened to the lateral side of the frame 1 for making it possible also to measure the profile from the lateral side.

A TOBEC-meter 15 is fitted to the rail 7 for the purpose of measuring the fat content directly on the body of the cow. The principle of operation is described in detail in British patent application GB1398735. There is provided a protecting element 16 around the TOBEC-meter 15 for protecting other electronic equipment against the electromagnetic radiation emitted by the meter. The TOBEC-meter 15 is suspended from the rail 7 for the purpose of being guided along the animal, independently of the animal or the robot arm 2.

There is provided a feed trough 17 for supplying feed. The controlling computer determines the desired amount and the desired type of feed that is supplied to the animal. A metal snout electrode 18 is provided in the feed trough 17. When the animal is consuming feed from the feed trough 17, its snout will make contact from time to time with the snout electrode 18. In a teat cup 3 there is provided a teat electrode for making contact with the teat. When the teat cup 3 has been connected, an alternating current can be applied over both electrodes. The animal will constitute a conductive connection between the snout electrode 18 and the teat electrode. A current meter subsequently measures the current intensity, after which the controlling computer determines the animal impedance (bio-electrical impedance, BIA) of the animal from the current intensity. Said animal impedance can be coupled to the total fat content of the animal. For this purpose, the controlling computer compares the measurement signal with values from a data table. In order to prevent leakage currents, the trough is electrically isolated from the frame 1.

In a non-shown embodiment, there is provided a drinking trough with water. An electrode is also provided in said drinking trough. When the cow puts her snout into the water to drink, the water will constitute the electrical connection between the snout and the electrode. This embodiment has the advantage that the water always makes a good contact with the snout.

The fat content signal that is obtained by means of BIA or TOBEC-measurement relates to the entire body or a major part of the body. For a good evaluation of said signal it is necessary to take data about inter alia the size of the foetus and the stage of pregnancy into account.

Figure 2 shows an embodiment, but not according to the invention, in which the profile of the rear part of the animal is determined by means of a set of feeler devices 19. These are disposed on a carrier beam so as to be situated side by side in the latitudinal direction. Via two cylinders 21, 22, said carrier beam 20 is movable in height as a whole relative to the frame 1. The feeler devices 19 are individually movable in height, such that they adapt themselves to the profile of the back of the animal. The height of each feeler device 19 is converted into an electrical measurement signal by a potentiometer that is fastened to the feeler device. The controlling computer processes the measurement signals from each feeler device 19 into a profile. The carrier beam 20 is movable as a whole in the longitudinal direction of the device by being guided over a rail 7. This makes it possible always to measure the most characterizing points of an animal for determining the body-score, in particular the cavities in the area in the vicinity of the tailhead, pin bone, thurl and hip bone of a cow, irrespective of the dimensions of the cow. For being guided smoothly over the back of the animal, the contact surface of a feeler device 19 consists of a rotatable element.

In a non-shown embodiment, instead of feeler devices, an ultrasonic meter is fastened to the carrier beam 20. By means of the cylinders 21 and 22, said ultrasonic meter is brought against the body of the cow, preferably in the vicinity of the hip bones. A good measurement requires a good contact with the skin. By means of picture analysis, the fat content is determined from the measured digital ultrasonic picture of the subcutaneous layers. The principle of this measurement is described in detail in U.S. patent publication US 5960105.

Figure 3 shows a device according to the invention for measuring the profile of the tail portion of a cow. The carrier beam 20 is provided with a bag 23 filled with a liquid, such as water. Via a flexible tube, said bag 23 is connected to a measuring glass 25 that is located at a higher level than the bag 23 and that is partially filled with the liquid. The carrier beam 20 is connected to cylinders 21, 22, so that the bag 23 can be moved in vertical direction. When an animal is present in the device, the bag 23 is brought from the position of rest above the animal onto the back of the animal. For this purpose, a stop 32 is fastened to the carrier beam, which stop 32 will rest on the spine of the animal. The stop 32 is designed as a roll, so that the animal can move easily in the longitudinal direction of the device relative to the profile meter. The bag 23 will assume a shape that is in accordance with the profile of the animal. According to the profile of the animal, the bag 23 will assume another shape and will have a greater or smaller contents, the so-called surface related contents. A smaller respectively a greater surface related contents will ensure that more respectively less liquid will be present in the measuring glass 25. The amount of liquid in the measuring glass 25 will thus form a measure for the profile and consequently for the fat content of the animal. Electrodes 26 are provided in the measuring glass 25 for determining the height of the liquid level 27. The controlling computer compares said measured height with data from a data table. Said data have been obtained from previous measurements and deviations therefrom give an indication of a change of the fat content. It is important that the measurements on the animal are carried out each time at exactly the same place. Due to the fact that the profile meter is coupled to the device 8 for following the rear of the animal, it is possible to measure each time at the same length co-ordinate. The stop 32 ensures that for each cow and for each measurement the same reference height is applied. There is also provided a flow meter 28 in the tube 24. By integration of the measured volume flow over the measurement time it is possible to determine the total change of volume.

There is provided a heating coil 26 in the bag for the purpose of heating the measuring liquid. This makes it possible to heat the liquid until it has reached a temperature that is pleasant to the animal. This also prevents the measuring liquid from freezing. Freezing can also be prevented by adding antifreeze to the liquid.

## Claims

1. A device provided with measuring means (11, 12, 13, 14, 15, 18, 19, 23) for a first animal related action comprising the automatic measurement of a characteristic of at least a part of the body of a living animal and for issuing a measurement signal, the device being provided with determining means for determining the fat content of at least a part of the body from the measurement signal, said measuring means (11, 12, 13, 14, 15, 18, 19, 23) comprising a profile meter for measuring at least a part of the profile of the animal, **characterized in that** the profile meter comprises a bag (23) to be filled with a fluid, which bag is provided with a fluid measuring device (31) for measuring the surface related contents of the bag (23).

2. A device as claimed in claim 1, **characterized in that** the profile meter comprises a stop for keeping the profile meter in a fixed position relative to the body of the animal.

3. A device as claimed in claim 1 or 2, **characterized in that** the fluid measuring device (31) comprises a volume meter (25).

4. A device as claimed in any one of the preceding claims, **characterized in that** the fluid measuring device (31) comprises a flow meter (28).

5. A device as claimed in any one of the preceding claims, **characterized in that** there is provided a heating element (26) in the fluid for keeping the fluid at a constant temperature.

6. A device as claimed in any one of the preceding claims, **characterized in that** the fluid is a liquid.

7. A device as claimed in any one of the preceding claims, **characterized in that** the device comprises means for automatically performing a second animal related action.

8. A device as claimed in any one of the preceding claims, **characterized in that** the device comprises an animal recognition device for recognising individual animals.

9. A device as claimed in any one of the preceding claims, **characterized in that** the determining means are provided with a processing device with a memory, the processing device comprising a data table for determining the fat content by means of the measurement signal and the processing device being suitable for issuing a fat content signal that is indicative of the determined fat content.

10. A device as claimed in claim 9, **characterized in that** the data table comprises data per group of animals.

11. A device as claimed in claim 9 or 10, **characterized in that** the data table comprises data per animal.

12. A device as claimed in claim 9, 10 or 11, **characterized in that** the processing device comprises a comparing device for comparing a momentary fat content determined from a momentary measurement signal with a historical fat content.

13. A device as claimed in any one of claims 9 to 12, **characterized in that** the device comprises an indicating device for indicating the fat content signal.

14. A device as claimed in any one of claims 9 to 13, **characterized in that** the device comprises a warning device for generating a warning, which warning device can be controlled by the fat content signal.

15. A device as claimed in any one of claims 9 to 14, **characterized in that** the device can be controlled by the fat content signal.

16. A device as claimed in any one of the preceding claims, **characterized in that** the device comprises a feeding installation for supplying feed.

17. A device as claimed in any one of the preceding claims, the animal being a dairy animal, **characterized in that** the device comprises a milking machine (30) for automatically performing on the dairy animal a milking related action, such as stimulation, foremilking, pre-cleaning, main milking, post-cleaning and the like.

18. A device as claimed in any one of the preceding claims, **characterized in that** the measuring means (11, 12, 13, 14, 15, 18, 19, 23) for measuring a characteristic of at least a part of the body of the animal and for issuing a measurement signal are measuring means for measuring an electromagnetic parameter of the animal.

19. A device as claimed in claim 18, **characterized in that** the measuring means comprise a total body electrical conductivity (TOBEC) meter (15).

20. A device as claimed in claim 18, **characterized in that** the measuring means comprise an electrical impedance meter.

21. A device as claimed in claim 20, **characterized in that** the device comprises a milking machine (30) for automatically performing on the dairy animal a milking related action, such as stimulation, foremilking, pre-cleaning, main milking, post-cleaning and the like, and **in that** the impedance meter comprises an udder electrode for contact with the udder of the animal.

22. A device as claimed in claim 21, **characterized in that** the udder electrode is disposed in or on a teat cup (3).

23. A device as claimed in claim 20, 21 or 22, **characterized in that** the impedance meter comprises a snout electrode (18) for contact with the snout of the animal.

24. A device as claimed in claim 23, **characterized in that** the device comprises a feeding installation for supplying feed and **in that** the snout electrode (18) is disposed in or on the feed trough (17) of the feeding installation.

25. A device as claimed in claim 1, **characterized in that** the profile comprises the profile of the upper side of the animal.

26. A device as claimed in claim 1 or 25, **characterized in that** the profile comprises the profile of the tail portion.

27. A device as claimed in claim 1, 25 or 26, **characterized in that** the profile comprises the profile of the shoulder.

28. A device as claimed in any one of claims 1, 25 to 27, c**haracterized that** the profile meter comprises a camera (11) that is connected to a picture-analysing device.

29. A device as claimed in any one of daims 1, 25 to 27, **characterized in that** the profile meter comprises a contactless telemeter (12, 13, 14) for measuring the distance from the meter to the surface of the animal.

30. A device as claimed in claim 29, **characterized in that** the telemeter comprises a laser meter.

31. A device as claimed in any one of the preceding claims, **characterized in that** the profile meter is connected to a following device (2, 8) for following movements of the animal.

32. A device as claimed in any one of the preceding claims, **characterized in that** the profile meter is movable relative to the animal.

## Patentansprüche

1. Anlage mit Meßvorrichtungen (11, 12, 13, 14, 15, 18, 19, 23) für eine erste tierbezogene Maßnahme zum automatischen Messen eines charakteristischen Merkmals zumindest eines Teiles des Körpers eines lebenden Tieres und zum Ausgeben eines Meßsignals, wobei die Anlage mit einer Ermittlungsvorrichtung zum Ermitteln des Fettgehaltes zumindest eines Teiles des Körpers anhand des Meßsignals versehen ist, wobei die Meßvorrichtungen (11, 12, 13, 14, 15, 18, 19, 23) ein Profilmeßgerät zum Vermessen zumindest eines Teiles der Oberfläche des Tieres umfassen,
**dadurch gekennzeichnet, daß** das Profilmeßgerät einen mit einem Fluid zu füllenden Beutel (23) umfaßt, wobei der Beutel mit einer Fluidmeßvorrichtung (31) zum Messen des oberflächenbezogenen Inhalts des Beutels (23) versehen ist.

2. Anlage nach Anspruch 1,
**dadurch gekennzeichnet, daß** das Profilmeßgerät einen Anschlag umfaßt, um das Profilmeßgerät in einer festen Position relativ zu dem Körper des Tieres zu halten.

3. Anlage nach Anspruch 1 oder 2,
**dadurch gekennzeichnet, daß** die Fluidmeßvorrichtung (31) einen Volumenmesser (25) umfaßt.

4. Anlage nach einem der vorhergehenden Ansprüche,
**dadurch gekennzeichnet, daß** die Fluidmeßvorrichtung (31) einen Durchflußmesser (28) umfaßt.

5. Anlage nach einem der vorhergehenden Ansprüche,
**dadurch gekennzeichnet, daß** ein Heizelement (26) in dem Fluid angeordnet ist, um das Fluid auf einer konstanten Temperatur zu halten.

6. Anlage nach einem der vorhergehenden Ansprüche,
**dadurch gekennzeichnet, daß** das Fluid eine Flüssigkeit ist.

7. Anlage nach einem der vorhergehenden Ansprüche,
**dadurch gekennzeichnet, daß** die Anlage Vorrichtungen zum automatischen Durchführen einer zweiten tierbezogenen Maßnahme umfaßt.

8. Anlage nach einem der vorhergehenden Ansprüche,
**dadurch gekennzeichnet, daß** die Anlage eine Tiererkennungsvorrichtung zum Erkennen einzelner Tiere umfaßt.

9. Anlage nach einem der vorhergehenden Ansprüche,
**dadurch gekennzeichnet, daß** die Ermittlungsvorrichtung eine Verarbeitungsvorrichtung mit einem Speicher umfaßt, wobei die Verarbeitungsvorrichtung eine Datentabelle zur Ermittlung des Fettgehaltes mit Hilfe des Meßsignals umfaßt und die Verarbeitungsvorrichtung geeignet ist, ein Fettgehaltsignal auszugeben, das den ermittelten Fettgehalt anzeigt.

10. Anlage nach Anspruch 9,
**dadurch gekennzeichnet, daß** die Datentabelle Daten für eine Gruppe von Tieren umfaßt.

11. Anlage nach Anspruch 9 oder 10,
**dadurch gekennzeichnet, daß** die Datentabelle Daten für jedes Tier umfaßt.

12. Anlage nach Anspruch 9, 10 oder 11,
**dadurch gekennzeichnet, daß** die Verarbeitungsvorrichtung eine Vergleichsvorrichtung zum Vergleichen eines aufgrund eines momentanen Meßsignals ermittelten momentanen Fettgehaltes mit einem historischen Fettgehalt umfaßt.

13. Anlage nach einem der Ansprüche 9 bis 12,
**dadurch gekennzeichnet, daß** die Anlage eine Anzeigevorrichtung zum Anzeigen des Fettgehaltsignals umfaßt.

14. Anlage nach einem der Ansprüche 9 bis 13,
**dadurch gekennzeichnet, daß** die Anlage eine Warnvorrichtung zur Erzeugung einer Warnung umfaßt, wobei die Warnvorrichtung von dem Fettgehaltsignal gesteuert werden kann.

15. Anlage nach einem der Ansprüche 9 bis 14,
**dadurch gekennzeichnet, daß** die Anlage von dem Fettgehaltsignal gesteuert werden kann.

16. Anlage nach einem der vorhergehenden Ansprüche,
**dadurch gekennzeichnet, daß** die Anlage eine Fütterungsvorrichtung zum Zuführen von Futter umfaßt.

17. Anlage nach einem der vorhergehenden Ansprüche, wobei das Tier ein milchgebendes Tier ist,
**dadurch gekennzeichnet, daß** die Anlage eine Melkmaschine (30) zum automatischen Durchführen einer melkbezogenen Maßnahme, wie z. B. Stimulieren, Vormelken, Vorreinigen, Hauptmelken, Nachreinigen und dergleichen, umfaßt.

18. Anlage nach einem der vorhergehenden Ansprüche,
**dadurch gekennzeichnet, daß** die Meßvorrichtungen (11, 12, 13, 14, 15, 18, 19, 23) zum Messen eines charakteristischen Merkmals zumindest eines Teiles des Körpers des Tieres und zum Ausgeben eines Meßsignals Meßvorrichtungen zum Messen eines elektromagnetischen Parameters des Tieres sind.

19. Anlage nach Anspruch 18,
**dadurch gekennzeichnet, daß** die Meßvorrichtungen einen Körperleitfähigkeitsmesser (TOBEC) (15) umfassen.

20. Anlage nach Anspruch 18,
**dadurch gekennzeichnet, daß** die Meßvorrichtungen einen Impedanzmesser umfassen.

21. Anlage nach Anspruch 20,
**dadurch gekennzeichnet, daß** die Anlage eine Melkmaschine (30) zum automatischen Durchführen einer melkbezogenen Maßnahme an einem milchgebenden Tier, wie z. B. Stimulieren, Vormelken, Vorreinigen, Hauptmelken, Nachreinigen und dergleichen, umfaßt, und daß der Impedanzmesser eine Euterelektrode zur Anlage an dem Euter des Tieres umfaßt.

22. Anlage nach Anspruch 21,
**dadurch gekennzeichnet, daß** die Euterelektrode in oder an einem Zitzenbecher (3) angeordnet ist.

23. Anlage nach Anspruch 20, 21 oder 22,
**dadurch gekennzeichnet, daß** der Impedanzmesser eine Schnauzenelektrode (18) zur Anlage an der Schnauze des Tieres umfaßt.

24. Anlage nach Anspruch 23,
**dadurch gekennzeichnet, daß** die Anlage eine Fütterungsvorrichtung zum Zuführen von Futter umfaßt, und daß die Schnauzenelektrode (18) in oder an dem Futtertrog (17) der Fütterungsvorrichtung angeordnet ist.

25. Anlage nach Anspruch 1,
**dadurch gekennzeichnet, daß** das Profil die Kontur der Oberseite des Tieres umfaßt.

26. Anlage nach Anspruch 1 oder 25,
**dadurch gekennzeichnet, daß** das Profil die Kontur des Schwanzabschnittes umfaßt.

27. Anlage nach Anspruch 1, 25 oder 26,
**dadurch gekennzeichnet, daß** das Profil die Kontur der Schulter umfaßt.

28. Anlage nach einem der Ansprüche 1, 25 bis 27,
**dadurch gekennzeichnet, daß** das Profilmeßgerät eine Kamera (11) umfaßt, die mit einer Bildanalysiervorrichtung verbunden ist.

29. Anlage nach einem der Ansprüche 1, 25 bis 27,
**dadurch gekennzeichnet, daß** das Profilmeßgerät einen kontaktfreien Entfernungsmesser (12, 13, 14) zum Messen der Entfernung zwischen dem Messer und der Oberfläche des Tieres umfaßt.

30. Anlage nach Anspruch 29,
**dadurch gekennzeichnet, daß** der Entfernungsmesser ein Lasermeßgerät umfaßt.

31. Anlage nach einem der vorhergehenden Ansprüche,
**dadurch gekennzeichnet, daß** das Profilmeßgerät mit einer Folgevorrichtung (2, 8) verbunden ist, die Bewegungen des Tieres folgt.

32. Anlage nach einem der vorhergehenden Ansprüche,
**dadurch gekennzeichnet, daß** das Profilmeßgerät relativ zu dem Tier bewegbar ist.

## Revendications

1. Dispositif doté de moyens de mesure (11, 12, 13, 14, 15, 18, 19, 23) pour une première action liée à un animal comprenant la mesure automatique d'une caractéristique d'au moins une partie du corps d'un animal vivant et destiné à émettre un signal de mesure, le dispositif étant doté de moyens de détermination destinés à déterminer la teneur en graisses d'au moins une partie du corps à partir du signal de mesure, lesdits moyens de mesure (11, 12, 13, 14, 15, 18, 19, 23) comprenant un profilomètre destiné à mesurer au moins une partie du profil de l'animal, **caractérisé en ce que** le profilomètre comprend un sac (23) à remplir d'un fluide, lequel sac est doté d'un dispositif de mesure de fluide (31) destiné à mesurer les contenus liés à la surface du sac (23).

2. Dispositif selon la revendication 1, **caractérisé en ce que** le profilomètre comprend un arrêt destiné à maintenir le profilomètre dans une position fixe par rapport au corps de l'animal.

3. Dispositif selon la revendication 1 ou 2, **caractérisé en ce que** le dispositif de mesure de fluide (31) comprend un volumètre (25).

4. Dispositif selon l'une quelconque des revendications précédentes, **caractérisé en ce que** le dispositif de mesure de fluide (31) comprend un débitmètre (28).

5. Dispositif selon l'une quelconque des revendications précédentes, **caractérisé en ce qu'**il est prévu un élément chauffant (26) dans le fluide destiné à maintenir le fluide à une température constante.

6. Dispositif selon l'une quelconque des revendications précédentes, **caractérisé en ce que** le fluide est un liquide.

7. Dispositif selon l'une quelconque des revendications précédentes, **caractérisé en ce que** le dispositif comprend des moyens destinés à effectuer automatiquement une seconde action liée à l'animal.

8. Dispositif selon l'une quelconque des revendications précédentes, **caractérisé en ce que** le dispositif comprend un dispositif de reconnaissance d'animal destiné à reconnaître les animaux individuels.

9. Dispositif selon l'une quelconque des revendications précédentes, **caractérisé en ce que** les moyens de détermination sont dotés d'un dispositif de traitement ayant une mémoire, le dispositif de traitement comprenant un tableau de données destiné à déterminer la teneur en graisses au moyen du signal de mesure et du dispositif de traitement qui est approprié pour émettre un signal de teneur en graisses qui est indicatif de la teneur en graisses déterminée.

10. Dispositif selon la revendication 9, **caractérisé en ce que** le tableau de données comprend des données par groupe d'animaux.

11. Dispositif selon la revendication 9 ou 10, **caractérisé en ce que** le tableau de données comprend des données par animal.

12. Dispositif selon la revendication 9, 10 ou 11, **caractérisé en ce que** le dispositif de traitement comprend un dispositif de comparaison destiné à comparer une teneur en graisses momentanée déterminée à partir d'un signal de mesure momentané à une teneur en graisses historique.

13. Dispositif selon l'une quelconque des revendications 9 à 12, **caractérisé en ce que** le dispositif comprend un dispositif d'indication destiné à indiquer le signal de teneur en graisses.

14. Dispositif selon l'une quelconque des revendications 9 à 13, **caractérisé en ce que** le dispositif comprend un dispositif d'avertissement destiné à générer un avertissement, lequel dispositif d'avertissement peut être commandé par le signal de teneur en graisses.

15. Dispositif selon l'une quelconque des revendications 9 à 14, **caractérisé en ce que** le dispositif peut être commandé par le signal de teneur en graisses.

16. Dispositif selon l'une quelconque des revendications précédentes, **caractérisé en ce que** le dispositif comprend une installation d'alimentation destinée à délivrer des aliments.

17. Dispositif selon l'une quelconque des revendications précédentes, l'animal étant un animal laitier, **caractérisé en ce que** le dispositif comprend une machine à traire (30) destinée à effectuer automatiquement sur l'animal laitier une action liée à la traite de lait, telle qu'une stimulation, des premiers jets, un pré-nettoyage, une traite principale, un post-nettoyage et similaires.

18. Dispositif selon l'une quelconque des revendications précédentes, **caractérisé en ce que** les moyens de mesure (11, 12, 13, 14, 15, 18, 19, 23) destinés à mesurer une caractéristique d'au moins une partie du corps de l'animal et destinés à émettre un signal de mesure sont des moyens de mesure destinés à mesurer un paramètre électromagnétique de l'animal.

19. Dispositif selon la revendication 18, **caractérisé en ce que** les moyens de mesure comprennent un appareil de mesure de la conductivité électrique corporelle totale (TOBEC) (15).

20. Dispositif selon la revendication 1, **caractérisé en ce que** les moyens de mesure comprennent un appareil de mesure d'impédance électrique.

21. Dispositif selon la revendication 20, **caractérisé en ce que** le dispositif comprend une machine à traire (30) destinée à effectuer automatiquement sur l'animal laitier une action liée à la traite de lait, telle qu'une stimulation, des premiers jets, un pré-nettoyage, une traite principale, un post-nettoyage et similaires, et **en ce que** l'appareil de mesure d'impédance comprend une électrode de mamelle pour un contact avec la mamelle de l'animal.

22. Dispositif selon la revendication 21, **caractérisé en ce que** l'électrode de mamelle est disposée dans ou sur un gobelet-trayeur (3).

23. Dispositif selon la revendication 20, 21 ou 22, **caractérisé en ce que** l'appareil de mesure d'impédance comprend une électrode de museau (18) pour un contact avec le museau de l'animal.

24. Dispositif selon la revendication 23, **caractérisé en ce que** le dispositif comprend une installation d'alimentation destinée à délivrer des aliments et **en ce que** l'électrode de museau (18) est disposée dans ou sur la caisse d'entrée (17) de l'installation d'alimentation.

25. Dispositif selon la revendication 1, **caractérisé en ce que** le profil comprend le profil du côté supérieur de l'animal.

26. Dispositif selon la revendication 1 ou 25, **caractérisé en ce que** le profil comprend le profil de la portion de queue.

27. Dispositif selon la revendication 1, 25 ou 26, **caractérisé en ce que** le profil comprend le profil de l'épaule.

28. Dispositif selon l'une quelconque des revendications 1, 2 à 27, **caractérisé en ce que** le profilomètre comprend une caméra (11) qui est connectée à un dispositif d'analyse d'image.

29. Dispositif selon l'une quelconque des revendications 1, 25 à 27, **caractérisé en ce que** le profilomètre comprend un appareil de télémesure sans contact (12, 13, 14) destiné à mesurer la distance de l'appareil de mesure à la surface de l'animal.

30. Dispositif selon la revendication 29, **caractérisé en ce que** l'appareil de télémesure comprend un appareil de mesure laser.

31. Dispositif selon l'une quelconque des revendications précédentes, **caractérisé en ce que** le profilomètre est connecté à un dispositif de suivi (2, 8) destiné à suivre les mouvements de l'animal.

32. Dispositif selon l'une quelconque des revendications précédentes, **caractérisé en ce que** le profilomètre peut être déplacé par rapport à l'animal.
